(19) Europäisches Patentamt
European Patent Office
Office européen des brevets

(11) Publication number: **0 439 208 A2**

# (12) EUROPEAN PATENT APPLICATION

(21) Application number: **91200038.7**

(22) Date of filing: **11.01.91**

(51) Int. Cl.[5]: **C12Q 1/68**, C12P 19/34, C07H 21/04, G01N 33/543

(30) Priority: **22.01.90 US 468456**

(43) Date of publication of application: **31.07.91 Bulletin 91/31**

(84) Designated Contracting States: **AT BE CH DE DK FR GB IT LI LU NL SE**

(71) Applicant: **EASTMAN KODAK COMPANY**
**343 State Street**
**Rochester, NY 14650-0221(US)**

Applicant: **CETUS CORPORATION**
**1400 Fifty-Third Street**
**Emeryville California 94608(US)**

(72) Inventor: **Wu, Annie Liu c/o EASTMAN KODAK COMPANY**
**Patent Department 343 State Street**
**Rochester New York 14650(US)**
Inventor: **Chang, Chu-an c/o EASTMAN KODAK COMPANY**
**Patent Department 343 State Street**
**Rochester New York 14650(US)**
Inventor: **Erlich, Henry Anthony, c/o EASTMAN KODAK COMPANY**
**Patent Department 343 State Street**
**Rochester New York 14650(US)**

(74) Representative: **Nunney, Ronald Frederick Adolphe et al**
**Kodak Limited Patent Department Headstone Drive**
**Harrow Middlesex HA1 4TY(GB)**

## (54) Method and kits for detecting human leukocyte antigen DNA.

(57) A rapid and simple method for detecting HLA DNA comprises amplifying HLA DNA using polymerase chain reaction in which at least one primer is biotinylated, contacting denatured amplified DNA strands with an oligonucleotide probe, and detecting the insolubilized materials using a peroxidase-avidin conjugate. The probe comprises a polymeric particle and an oligonucleotide which is complementary to the amplified product, is 15 to 25 nucleotides in length, and is attached to the particle through a linking group having 16 to 30 ethylene glycol units. This method is used to advantage to type HLA, or to detect HLA DNA in a specimen. The materials needed to carry out the method can be provided in test kits. Desirably, the assay is carried out using a disposable test device having a microporous filtration membrane.

EP 0 439 208 A2

# METHOD AND KITS FOR DETECTING HUMAN LEUKOCYTE ANTIGEN DNA

This invention relates to human genotyping. More specifically, the invention concerns a method for HLA DNA detection using polymerase chain reaction and a disposable test device for detection. HLA typing is a preferred use of the method. Forensic testing is one application of the present invention. It also relates to test Kits useful in practicing the method of this invention.

As the major constituent of a human chromosome, DNA carries the genetic code which determines each person's individual characteristics (such as eye color, Skin pigmentation, hair texture). Thus, the DNA sequences are unique for individuals. Forensic DNA methods are based on the fact that no two individuals have exactly the same DNA sequence.

The major histocompatibility complex of humans is a cluster of genes occupying a region located on the sixth chromosome. This complex, designated the Human Leukocyte Antigen (identified herein as HLA), has been divided into six major gene loci, now designated as HLA-A, HLA-B, HLA-C, HLA-DP. HLA-DQ and HLA-DR. The A, B And C loci encode the classical transplantation antigens, whereas the DP, DQ and DR loci encode products that control immune responsiveness. Further details of the genetics and biology of HLA are found in EP-A-0 103 960 and references cited therein.

The products encoded by the HLA loci have been typed serologically or by mixed lymphocyte culture methods. Such typing is commonly used in paternity determinations, transplant and transfusion compatibility testing, blood component therapy, anthropological studies and in disease association correlation to diagnose diseases or predict susceptibility to diseases. The major drawbacks of such typing are the complexity of blood sera and the lack of the widespread availability of standard sera necessary to conduct such tests.

Forensic testing is normally separated into two different categories:

(1) non-criminal investigations, such as paternity testing or immigration evaluations, which test relatively fresh biological specimens, and

(2) criminal investigations for identifying persons from blood, hair or semen samples, in which the age, condition and size of the sample may be critical to the results, and contamination is a potential serious problem.

In all of these instances, DNA in the sample is compared to the DNA of another sample. For example, in paternity tests, the DNA of the alleged father is compared to the child's DNA.

Many known methods of HLA typing are based on restriction fragment length polymorphism which require high molecular weight DNA, that is DNA which has not been degraded. However, degradation can occur at the scene of a crime or while in storage. Thus, such specimens may not provide results reliably, especially for criminal investigations. These procedures also require relatively large specimens, and are tedious and time consuming. More details about such methods are provided by Connor, New Scientist, January 28, 1988 and in US-A-4,582,788. Conventional dot blotting or solution hybridization are used in these instances to detect the HLA gene sequences present in the specimens.

Another procedure for HLA typing has been described by Saiki and others, Nature, 324, pp. 163-166 (1986) in which a specific segment of the HLA-DQ$\alpha$ gene is amplified using polymerase chain reaction (PCR) prior to detection using allele-specific oligonucleotide probes which are labeled with a radioisotope or biotin. Detection was achieved using a nylon filter as a "dot blot".

It would be desirable to have a method for HLA typing or detection which is simple to use and rapid and which can also be used for diagnostic assays using simple disposable test devices.

The problems of known methods are overcome, and a number of important advantages are provided with a method for detecting HLA DNA comprising the steps of:

A. amplifying HLA DNA strands in a specimen using a polymerase chain reaction and two primers complementary to the DNA strands, at least one of which primers is biotinylated, to form biotinylated primer extension products complementary to one of the strands,

B. denaturing the amplified DNA strands and primer extension products in admixture,

C. contacting the mixture with at least one oligonucleotide probe comprising a polymeric particle and an oligonucleotide, the oligonucleotide being

(i) complementary to the biotinylated primer extension product,

(ii) 15 to 25 nucleotides in length, and

(iii) attached to the particle through a linking group having 16 to 30 ethylene glycol units

to form an insoluble hybrid of the probe and biotinylated primer extension product,

D. separating the insoluble hybrid from soluble materials,

E. contacting the insoluble hybrid with a peroxidase-avidin conjugate to form a reaction product of

biotinylated primer extension product and the conjugate,

F. contacting the reaction product with a composition which provides a dye in the presence of hydrogen peroxide and peroxidase, and

G. determining the dye formed in step F as a measure of the presence of HLA DNA in the specimen.

A kit for detecting HLA DNA comprises:

a. a oligonucleotide probe comprising a polymeric particle and an oligonucleotide, and

b. a composition for providing a dye in the presence of peroxidase and hydrogen peroxide,

the kit characterized wherein the oligonucleotide is

(i) complementary to a HLA DNA strand,

(ii) 15 to 25 nucleotides in length, and

(iii) attached to the particle through a linking group having 16 to 30 ethylene glycol units.

Moreover, a different kit for detecting HLA DNA comprises:

a. a oligonucleotide probe comprising a polymeric particle and an oligonucleotide, and

b. an avidin-peroxidase conjugate,

the kit characterized wherein the oligonucleotide is

(i) complementary to a HLA DNA strand,

(ii) 15 to 25 nucleotides in length, and

(iii) attached to the particle through a linking group having 16 to 30 ethylene glycol units.

This invention also provides for the detection of HLA DNA using a oligonucleotide probe comprising a polymeric particle and an oligonucleotide, the oligonucleotide being

(i) complementary to the biotinylated primer extension product,

(ii) 15 to 25 nucleotides in length, and

(iii) attached to the particle through a linking group having 16 to 30 ethylene glycol units.

The present invention provides a rapid, simple and sensitive method for typing HLA DNA fragments, or detecting for HLA DNA in a test specimen, and kits for accomplishing these results. The method is sensitive because the probe used is highly efficient in hybridizing with the amplified PCR products. This result is achieved by using a probe which has 15 to 25 nucleotides which is complementary to the HLA DNA strand of interest. Moreover, the oligonucleotide is connected to a polymeric particle with a specific linking group having 16 to 30 ethylene glycol units. The method is simplified and easy to use because the insolubilized products are easily and rapidly separated from soluble materials, preferably using a microporous filtration membrane preferably mounted in a disposable test device. Smaller test specimens can be evaluated with this method. Moreover, the invention can easily be used by crime laboratory personnel as opposed to sending the specimen to a clinical test center.

The present invention is directed to the detection of one or more specific nucleic acid sequences present in HLA DNA present in a biological test specimen. Preferably, the method is used for HLA genotyping, but detection of an unknown DNA is also desirable in certain instances. In the following discussion, genotyping will be predominant in the description, but it will be understood by one skilled in the art that the method can be adapted to detection procedures and needs other than HLA genotyping.

The initial step in typing a person's HLA is to obtain a sample of DNA from that person. As used herein, the term "person" also includes preborn humans as well as deceased persons. All nucleated cells contain genomic DNA and, therefore, are potential sources for the required nucleic acids. HLA DNA can be obtained and detected in hair samples, semen, blood or components thereof containing cellular material, tissues, saliva containing cellular material, amniotic fluid and other human specimens readily apparent to one skilled in the art. The amount of specimen needed will vary with the type of specimen.

Genomic DNA is isolated from the cells in a specimen using any suitable technique, many of which are well known in the art, as long as the technique does not degrade the DNA. For example, it can be extracted from cells using a buffered proteinase K composition at a temperature and pH which reduces the likelihood that the DNA will be degraded, as described, for example by Kan and others, New Eng.J.Med., 297, pp. 1080-1084 (1977) and in Nature, 251, pp. pp. 392-393 (1974). Other extraction techniques involving phenol or other extraction solvents are described in EP-A-0 145 356, EP-A-0 240 191, and EP-A-0 245 945.

In a preferred extraction procedure, a specimen is heated at or near 100 °C for a few minutes to lyse cells and release nucleic acids, followed by centrifugation or another isolation technique. In a second procedure, proteinase K is used in a buffered composition containing PCR reagents, followed by heating for an hour at 50 °C and boiling to deactivate the enzyme.

The extracted DNA can be used as is or purified by dialysis, chromatography, affinity capture using complementary nucleic acids or by any other suitable technique readily apparent to one skilled in the art.

Once isolated DNA is obtained, it is subjected to an amplification procedure which results in exponential amplification of the molecule relative to the number of steps involved. It is to be understood that where

several DNA sequences are of interest, the amplification process can be used to multiply all of them. The end result is a large number of detectable nucleic acids which can then be subjected to typing procedures for evaluation.

The amplification process generally used is described in considerable detail in US-A-4,683,195 and US-A-4,683,202. That process will be only summarized herein because it is becoming widely known in the art. Example 1 below illustrates the practice of the amplification procedure.

As used herein in referring to primers or probes, the term "oligonucleotide" refers to a molecule comprised of two or more deoxyribonucleotides or ribonucleotides, and preferably more than three. The exact size is not critical (except for the probe described below) but depends upon many factors including the ultimate use or function of the oligonucleotide. The oligonucleotide may be derived synthetically or by other methods known in the art.

The term "primer" refers to an oligonucleotide, whether naturally occurring or synthetically produced, which is capable of acting as a point of initiation of synthesis when placed under conditions in which synthesis of a primer extension product complementary to a nucleic acid strand is induced. Such conditions include the presence of nucleotides (such as the four standard deoxyribonucleoside triphosphates) and an agent for polymerization such as a DNA polymerase, and suitable temperature and pH.

A mixture of primers can be used in the amplification, and a set of primers is generally used for each nucleic acid sequence to be amplified. Each primer is substantially complementary to a nucleic acid sequence of the target DNA. By "substantially complementary" is meant that there are a sufficient number of bases on the primer that match with the corresponding bases in the target DNA that the primer will hybridize with that sequence. It does not mean, however, that every base pair will match.

The primers are generally single-stranded. The exact size of each primer will vary depending upon the use contemplated, the complexity of the target sequence, reaction temperature and the source of the primer. Generally, the primers used in this invention will have from 15 to 50 nucleotides, and preferably, they have from 20 to 30 nucleotides.

Primers useful herein can be obtained from a number of sources or prepared using known techniques and equipment, including for example, an ABI DNA Synthesizer (available from Applied Biosystems) or a Biosearch 8600 Series or 8800 Series Synthesizer (available from Milligen-Biosearch, Inc.) and known methods for their use. Naturally occurring primers isolated from biological sources are also useful (such as restriction endonuclease digests).

At least one of the primers used in the method is biotinylated, that is, it has a biotin moiety covalently attached thereto. Such conjugates of primer and biotin can be readily prepared using known technology, described for example in by Connolly in Nucleic Acids Research, 15(7), 3131 (1987). A preferred procedure for biotinylating a primer is described in WO-A-89/02931.

Once genomic DNA has been extracted, its denatured strands are contacted with suitable primers under conditions such that a mixture of hybridized products of primers and target DNA strands are formed. Such conditions are those normally used for amplification as described in US-A-4,683,202. Then, primer extension products are formed with at least one of the hybridized products followed by additional priming and extension product formation. After denaturation (that is, separation of complementary products), the replicated target nucleic acid can be isolated from the reaction mixture using standard procedures and equipment. At least one of the replicated nucleic acids is biotinylated and useful in later steps of the method.

The present invention is also useful for detection of a specific nucleic acid having two complementary strands. Most nucleic acid sequences of interest already are double-stranded, such as those found in DNA. However, single-strand nucleic acid sequences, such as mRNA, can be similarly detected after it is converted to a double-stranded sequence using reverse transcriptase.

A specific nucleic acid sequence is reproduced using the nucleic acid containing that sequence as a template. If the nucleic acid contains two strands, it is necessary to separate the strands, either as a separate step or simultaneously with the formation of primer extension products. Denaturing can be accomplished using any suitable physical, chemical or enzymatic means as described in the art. Heating to a suitable temperature is a preferred means.

Once the separated strands are available for use, synthesis of additional nucleic acid strands can be carried out using the primers in a buffered aqueous solution generally at a pH of from 7 to 9. Preferably, a molar excess of the primers is added to the buffered solution, and specific amounts are taught in the art (for example, US-A-4,683,202). The deoxyribonucleoside triphosphates dATP, dCTP, dGTP and dTTP are also added to the synthesis mixture in adequate amounts and the resulting solution is heated to 90-100 °C for up to 10 minutes, and preferably from 1 to 4 minutes. After this heating, the solution is preferably cooled to room temperature, and an appropriate agent for inducing (or catalyzing) the formation of primer extension

products is introduced. This inducing agent is generally known in the art as a polymerization agent. Reaction to form these products is carried out under known conditions (generally from room temperature to that temperature at which polymerization no longer occurs).

The polymerization agent may be any compound, or combination of reagents, which will function to accomplish the synthesis of primer extension products, including enzymes (for example, E. coli DNA polymerase I, T4 DNA polymerase, Klenow polymerase, reverse transcriptase and others known in the art). Particularly useful enzymes are thermally stable enzymes, cloned or naturally occurring, such as those obtained from various Thermus bacterial species. Other polymerization agents are described in US-A-4,683,202.

Preferred thermal-stable enzymes are DNA polymerases from Thermus aquaticus as described in EP-A-O 258 017. Other useful enzymes are described by Rossi and others, Syst. Appl. Microbiol. 2(2-3), pp. 337-341, 1986. Some useful polymerases are commercially available. Generally, the synthesis of extension products will be initiated at the 3' end of each primer and proceed in the 5' to 3' direction along the template until synthesis is terminated. Some polymerization agents (for example, reverse transcriptase) may proceed in the 3' to 5' direction along the template.

The newly formed primer extension products comprising the newly synthesized strands and their respective primers form double-stranded molecules with the initial target strands which are used in the succeeding steps of the method. These strands are then separated by denaturation as described above to provide single-stranded molecules, onto which new nucleic acids are synthesized as described above. Additional reagents may be needed to beep the amplification procedure going, after which most of the extension products will consist of the specific nucleic acid sequence bounded by the primers (that is, complementary products).

The steps of strand separation and extension product synthesis can be repeated as often as needed to produce the desired quantity of the specific nucleic acid needed for the use, for example detection. Generally, the sequence of steps is repeated at least once, and preferably at least 10 to 50 times.

when it is desired to produce more than one specific nucleic acid from the first nucleic acid or a mixture thereof, the appropriate number of sets of primers are used in the general procedure described above.

Generally, once a desired amount of the nucleic acid sequence of interest has been generated and the primer extension products are separated for a last time, the primer extension products (including the biotinylated ones) are contacted with an oligonucleotide probe defined herein for capturing the products for later separation. The probe has a nucleic acid sequence (that is, oligonucleotide) containing 15 to 25, and preferably 17 to 20, nucleotides which is complementary to the nucleic acid sequence of the biotinylated primer extension product, that is the product formed from the biotinylated primer. This probe and the biotinylated primer extension product then form an insoluble hybrid which can be separated from soluble materials as described below.

Moreover the probe oligonucleotide is attached to a polymeric particle through a linking group having 16 to 30, and preferably 16 to 24, ethylene glycol units. The details for preparing and using such linking groups are provided in WO-A-89/02931.

The oligonucleotide is attached to the polymeric particle through the noted sequence of ethylene glycol units, preferably in a sequence having a multiple of four units, such as 16, 20, 24 and 28 units. In general, this is achieved by preparing the oligonucleotide using standard solid phase phosphoramidite chemistry. An aminotetraethylene glycol is prepared and attached to the 5' end of the oligonucleotide. The reactive group, for example a carboxy group, of the polymeric particle is then reacted with the aminotetraethylene glycol using an activating agent, such as a carbodiimide or onium salt, as is known in the art, to attach the probe to the particle. These procedures are generally described in WO-A-89/02932 and in Oligonucleotide Synthesis: A Practical Approach (M.J. Gait, Ed., IRL Press, Oxford, U.K., 1984).

Useful polymeric particles for preparing the probe are composed of any suitable polymeric material which is water-insoluble, and which provides the necessary surface reactive groups for attaching the oligonucleotide through the linking group. Thus, the surface reactive groups may be, for example, sulfhydryl, amino, active halogen atoms, epoxy groups, isocyanate, aziridine, activated 2-substituted ethylsulfonyl, vinylsulfonyl, active aldehyde, 2-substituted ethylcarbonyl, carboxy and others which will react with the respective sulfhydryl, amino or hydroxy groups of the linking group.

The polymeric particles can be of any useful size needed for the assay as long as they will not pass through the microporous membrane used in the separation step (described below). Generally, they average at least 0.1 μmeters in diameter, with particles of from 0.2 to 5 μmeters average diameter being preferred.

The particles can be composed of any suitable condensation or addition polymer, with the latter polymer formed from one or more ethylenically unsaturated polymerizable monomers being preferred. In preparing these polymers by emulsion or suspension polymerization techniques, at least one of the

monomers has the requisite reactive groups. The particles can be composed of the same polymer throughout, or they can be core-shell particles as described, for example, in US-A-4,703,018 and EP-A-0 280 556. The shell polymer of core-shell particles have the requisite reactive groups.

Particularly useful polymers are prepared from monomers, at least one of which has a reactive carboxy group, or monomers which will provide such groups upon chemical treatment such as hydrolysis of anhydrides or oxidation of surface methylol or aldehyde groups. Useful monomers include, but are not limited to acrylic acid, methacrylic acid, itaconic acid, aconitic acid, fumaric acid, maleic acid, B-carboxyethyl methacrylate, m & p-carboxymethylstyrene, methacrylamidohexanoic acid and N-(2-carboxy-1,1-dimethylethyl)acrylamide or a salt or anhydride precursor thereof.

One or more of the monomers described above can be polymerized individually or in combination to form home or copolymers. Preferably, one or more of them are copolymerized with at least one other ethylenically unsaturated polymerizable monomer. Preferred polymers can be represented by the formula (I):

$$-(A)_x-(B)_y-(D)_z- \qquad (I)$$

wherein -A- represents recurring units derived from one or more hydrophobic ethylenically unsaturated monomers,

-B- represents recurring units derived from one or more ethylenically unsaturated monomers having the requisite reactive groups described above, and

-D- represents recurring units derived from one or more ethylenically unsaturated monomers which are different than those represented by -A- or -B-.

In formula (I), x is from 0 to 99.9 mole percent, y is from 0.1 to 100 mole percent, and z is from 0 to 20 mole percent. Preferably, x is from 45 to 99.5 mole percent, y is from 0.5 to 50 mole percent, and z is from 0 to 10 mole percent.

Monomers from which the -A- recurring units are derived, both in general and in preferred embodiments, are hydrophobic and form homopolymers that are insoluble in water. Preferably, these monomers have aromatic groups. Representative hydrophobic monomers include, but are not limited to, styrene and styrene derivatives (for example, 4-vinyltoluene, 2,5-dimethylstyrene, 4-t-butylstyrene, 2-chlorostyrene and others known in the art), acrylic and methacrylic acid esters and amides (for example, n-butyl acrylate, propyl methacrylate, methyl acrylate, methyl methacrylate, ethyl methacrylate, 2-ethylhexyl methacrylate, N-phenylacrylamide and others known in the art), acrylonitrile and vinyl acetate.

This polymer can be crosslinked, if desired, in any suitable fashion. One method is to incorporate a small amount, that is up to 10 mole percent, and preferably from 0.3 to 5 mole percent, of a monomer having two or more ethylenically unsaturated polymerizable groups. These monomers are included among the hydrophobic monomers from which A is derived. Representative monomers are described in Research Disclosure, publication 19551, July, 1980, page 304, and include for example, divinylbenzene, ethylene dimethacrylate, N,N'-methylenebisacrylamide, 2,2-dimethyl-1,3-propylene diacrylate, allyl acrylate, ethylidyne trimethacrylate and ethylene diacrylate.

Preferred monomers from which the -A- recurring units are derived are vinyl aromatic monomers, especially styrene and styrene derivatives.

The monomers from which the -B- recurring units are derived are those having the reactive groups described above.

Monomers from which the -D- recurring units are derived include monomers different than those from which -A- and -B- are derived. Specifically, the -D- recurring units are derived from monomers which impart aqueous dispersion stability to the particles or other properties. Representative monomers include, but are not limited to, anionic monomers such as sodium 2-acrylamido-2-methylpropanesulfonate, styrene sulfonic acid, potassium salt and other ethylenically unsaturated polymerizable sulfonates, sulfates and phosphonates, other hydrophilic but nonionic monomers, such as 2-hydroxyethyl acrylate and 2-hydroxyethyl methacrylate and others known to one skilled in the art.

Preferred monomers from which the -D- units are derived are sodium 2-acrylamido-2-methylpropanesulfonate, and p-styrenesulfonic acid, potassium salt.

Representative polymers of the monomers described above include the following:
poly(styrene-co-acrylic acid)(90:10 molar ratio), poly(m & p-chloromethylstyrene), poly(styrene-co-m & p-chloromethylstyrene-co-2-hydroxyethyl acrylate) (67:30:3 molar ratio), poly[styrene-co-m & p-(2-chloroethylsulfonylmethyl)styrene] (96:4 molar ratio), poly{styrene-co-N[m & p-(2-chloroethylsulfonylmethyl)phenyl]-

acrylamide} (99.3:0.7 molar ratio), poly(m & p-chloromethylstyrene-co-methacrylic acid)(95:5, 98:2 and 99.8:0.2 molar ratio), poly[styrene-co-m & p-(2-chloroethylsulfonylmethyl)styrene-co-methacrylic acid]-(93.5:4.5:2 molar ratio), poly[styrene-co-N-[m & p-(2-chloroethylsulfonylmethyl)phenyl]acrylamide-co-methacrylic acid) (97.3:0.7:2 molar ratio), poly(styrene-co-methacrylic acid)(90:10 molar ratio), poly(styrene-co-acrylic acid)(60:40 molar ratio), poly(styrene-co-acrylic acid-co-hydroxyethyl methacrylate)(85:10:5 molar ratio) and poly(styrene-co-m & p-chloromethylstyrene)(70:30 molar ratio).

The polymers described above are prepared using known polymerization processes, including emulsion and suspension techniques, which are explained in numerous textbooks and publications.

As noted above, the insoluble hybridized product of probe and biotinylated primer extension product are separated from soluble materials. Separation can be accomplished using various known means, such as filtration, settling of insoluble materials, but preferably a microporous filtration membrane is used. Particularly useful microporous filter membranes include polyamide membranes marketed by Pall Corp. (for example as Ultipore™, LoProdyne™ or Biodyne™ membranes). They can be used uncoated or precoated with surfactants or other materials which reduce nonspecific interactions, such as succinylated casein.

The membranes can be used as a separate substrate with suitable containers for collecting fluid and soluble materials. Most preferably, however, they are mounted as part of a test device. Various test devices are known in the art including those described in US-A-3,825,410, US-A-3,888,629, US-A-3,970,429 and US-A-4,446,232. Particularly useful devices are described in EP-A-0 308 231.

The insoluble hybrid collected on the microporous membrane is contacted with a peroxidase-avidin conjugate which complexes with the biotinylated portion of the hybrid. Useful conjugates are commercially available from a number of sources. It usually requires a few minutes for the complexation to occur and uncomplexed conjugate passes through the membrane along with any fluid.

Following this, the reaction product on the membrane is contacted with a composition which provides a dye in the presence of hydrogen peroxide and peroxidase. This composition can include one or more reagents to provide the dye, and can optionally include hydrogen peroxide although the peroxide can be added separately.

Useful dyes or dye-providing reagents are known in the art. For example, useful dye-providing reagents include tetramethylbenzidine such as described in WO-A-88/02806 and WO-A-88/02807 and derivatives thereof, and leuco dyes, such as triarylimidazole leuco dyes (as described in US-A-4,089,747). Particularly useful dye-providing compositions are triarylimidazole leuco dyes used in combination with certain stabilizing materials. A representative composition is shown in Example 1 below.

The presence of the dye on the filtration membrane is an indication of the presence of HLA DNA sequence in the specimen tested. The dye can often be observed visually without the use of equipment, but in some instances, the dye density may be faint or outside the visible region of the electromagnetic spectrum, and thus require suitable detection equipment (that is, spectrophotometers) for adequate determination. Procedures for doing this are well known in the art.

The method described herein can be used to provide the detection or characterization of specific nucleic acid sequences associated with any locus of HLA, but particularly those associated with the HLA-DQα locus. This characterization can be useful in detected genetic disorders, genetic patterns, forensic investigations, paternity tests, and other instances known in the art.

A kit of this invention comprises the oligonucleotide probe described above as well as a dye-providing composition. In another embodiment, a different kit includes the probe and an avidin-peroxidase conjugate. In addition, each kit can include the additional reagents and equipment needed for the method or such materials can be supplied individually or in combinations. For example, such materials include a disposable test device containing a membrane or separate microporous filtration membrane, a avidin-peroxidase conjugate, primers, a dye-providing composition, deoxyribonucleoside triphosphates, buffers, wash solutions, pipettes, instructions and any other useful equipment or materials. The kits can also include a multiplicity of probes which will collectively hybridize to all alleles of the HLA gene.

In a preferred embodiment for HLA typing, several sets of primers and probes can be used to detect different loci of HLA, and the evaluation can be made on a single or multiple membranes (in single or multiple test devices). If a single membrane is used, the multiple detection of loci can be done in different regions thereof, perhaps by depositing different specific probes in those regions.

The following examples are used to illustrate the practice of this invention, but are not meant to be limiting in any way. All percentages are by weight unless otherwise identified.

Example 1: Detection of HLA-DQα DNA

This example illustrates the practice of the method of this invention to detect HLA-DQα locus in a test

specimen after purification and amplification of the target DNA.

Materials:

The compound 1-(3-dimethylaminopropyl)-3-ethylcarbodiimide hydrochloride is used to identify the carbodiimide used for attaching the oligonucleotide to the carboxy groups of the particles.

An avidin-horseradish peroxidase conjugate was purchase from Sigma Chemical Co.

A DNA polymerase was used which was isolated from Thermus aquaticus having an activity of about 4 I.U./μl.

The leuco dye composition used below contained the following components: 2-(4-hydroxy-3,5-dimethoxyphenyl)-4,5-bis(4-methoxyphenyl)imidazole(0.008%), poly(vinyl pyrrolidone)(1%), hydrogen peroxide (10 mmolar), 4'-hydroxyacetanilide (5 mmolar), diethylenetriaminepentaacetic acid (10 μmolar) in sodium phosphate buffer (10 mmolar, pH 6.8).

Two HLA homozygous cell lines HAR and LBF (obtained from the National Institute of General Medical Sciences Human Genetic Mutant Cell Repository, Coriell Institute for Medical Research, Camden, New Jersey) were evaluated. The first was used as a Target segment to be detected, and was complementary to the probe (described below). A second segment was used as a Control, and was not complementary to the probe. The amplified Target segment contained 242 nucleotides.

The probes used in the assay comprised an aminooligonucleotide having the following sequence:

SEQ ID NO: 1        5'-N-CTGGTGTTTG CCTGTTCTC-3'

wherein N represents an aminotetraethyleneglycol linking arm having 16 ethylene glycol units for use in the assay of this invention. A Control probe having the same sequence but no X group was also used. The oligonucleotide-linking arm conjugate having the ethylene glycol units was prepared using the procedure described in WO-A-89/02931.

Latex particles used (2 μm, 2.9% solids) were comprised of poly(styrene-co-acrylic acid)(97.5:2.5 molar ratio).

An SSPE solution (2%) contained sodium chloride (43.5 g), sodium phosphate (0.276 g), ethylenediaminetetraacetic acid (0.074 g ) in 1 liter of water, having the pH adjusted to 7.4.

The primers used in the amplification procedure had the following nucleic acid sequences:

SEQ ID NO: 2
Primer 1: 5'-N'-GTGCTGCAGG TGTAAACTTG TACCAG-3'
SEQ ID NO: 3
Primer 2: 5'-N'-CGGATCCGGT AGCAGCGGTA GAGTTG-3'

wherein N' represents a biotintetraethylene glycol linker arm prepared and attached using the procedure described in WO-89/02931 (noted above).

Probe Preparation:

The oligonucleotide probe was prepared in the following manner. An aqueous latex solution (1 ml, 30 mg beads) was centrifuged (2.5 minutes at 13,000 times gravity) to remove the supernatant. The beads were then resuspended in glass-distilled water (1 ml) by vigorous vortexing. The mixture was again centrifuged, the supernatant removed, and the beads were resuspended in a buffer solution (1 ml, containing 0.2 molar methylimidazole and 3 normal sodium chloride, pH 7).

To the bead solution was added the 5'-aminooligonucleotide described above (2.5 nmoles), and the resulting solution was mixed well. The compound 1-(3-dimethylaminopropyl)-3-ethylcarbodiimide hydrochloride (5 mg) was added. After thorough mixing by vortex, the reaction mixture was left at 20-25 °C with occasional mixing for at least 2 hours (usually for 15 hours).

The supernatant was first removed by centrifugation and the beads were subsequently washed with the following fluids by centrifugation and pipetting off the supernatant between solutions:

a. three times with 1 ml glass-distilled water, and

b. three times with a 10% SSPE buffer solution (further containing 0.5% dodecyl sulfate, prewarmed to 70 °C).

After the last treatment, the beads were resuspended in glass-distilled water to a final volume of 1 ml to make a 3% bead suspension and kept at 4 °C.

Amplification:

Both the Target and Control DNA segments were amplified separately in the following manner.

To a buffered solution (100 μl) containing tris(hydroxymethyl)aminomethane hydrochloride (67 mmolar, pH 8.8), ammonium sulfate (16.6 mmolar), magnesium chloride (2.5 mmolar) and gelatin (10 μg) were added the primers described above (20 pmoles of each), the dNTPs (0.175 mmolar of each), DNA polymerase identified above (12 units) and the DNA segments noted above. Amplification was carried out for 35 consecutive cycles as follows:

| | |
|---|---|
| 70°C rising to 95°C | 1 minute |
| 95°C | 0.5 minute (denature) |
| 95°C lowering to 50°C | 1.25 minutes |
| 65°C | 0.5 minute (hybridize) |
| 65°C rising to 70°C | 0.75 minute |
| 70°C | 1 minute (extend primers) |

Samples (10 μl) of both Target and Control amplified HLA DNA sequences were heated to 95 °C for five minutes to denature the hybridized products, then mixed with the insoluble probe described above (150 μg) in a buffer solution (0.125 molar sodium phosphate, 2.5 normal sodium chloride and 1 mmolar ethylenediaminetetraacetic acid, pH 6.8). The resulting mixture was incubated at 42 °C for 10 minutes to allow the amplified biotinylated DNA strands to hybridize to the probe.

The hybridized products for each segment was added to individual Surecell™ disposable test devices (Eastman Kodak Co.) containing a LoProdyne™ polyamide microporous membrane (1.2 μm, Pall Corp.), and fluid was allowed to flow through the membrane. The hybridized product of probe and biotinylated DNA strands was retained on the surface of the membrane.

This insoluble material was washed with 10% SSPE buffer containing sodium dodecyl sulfate (0.5 %) which had been previously warmed to 55 °C. An avidin-horseradish peroxidase conjugate (15 μl) was added to each disposable test device followed by incubation at 20-25 °C for 2 minutes. The insoluble product was again washed to remove uncomplexed conjugate, and the leuco dye composition noted above (100 μl) was then added to each test device. After further incubation at 20-25 °C for 5 minutes, the resulting color was visually graded against a color chart with zero representing no color and 10 representing the highest density. The results, shown in the following Table as reflectance density ($D_R$), indicate that the probe used in the assay of the invention provided considerably higher color formation than the Control assay.

TABLE

| HLA Cell Line | Probe of This Invention | Control Probe |
|---|---|---|
| HAR (complementary to probe) | 10 | 5.5 |
| LBF (not complementary) | 1 | 1 |
| Blank | 1 | 1 |

Example 2: Detection of Genomic DNA from Human Hair

This example illustrates the practice of this invention to detect HLA DNA found in human hair.

Three pieces of hair obtained from the head of a volunteer were washed with distilled water, followed by washing with ethanol (about 5 ml). After drying the hair at room temperature, the hair was digested in a solution (0.5 ml) comprising tris(hydroxymethyl)aminomethane hydrochloride (25 mmolar, pH 7.6), ethylenediaminetetraacetic acid (10 mmolar), sodium dodecyl sulfate (2 %), dithiothreitol (40 mmolar) and proteinase K (150 $\mu$g) at 56 °C for about 15 hours. The DNA was extracted with 0.5 ml of a 50:50 volume mixture of phenol and chloroform, followed by treatment with n-butanol following the procedure of Maniatis and others, Molecular Cloning, pp. 458-459, 1982. DNA was recovered in 250 $\mu$l of a buffer solution containing tris(hydroxymethyl)aminomethane hydrochloride (10 mmolar) and ethylenediaminetetraacetic acid (1 mmolar) having the pH adjusted to 8 with hydrochloric acid. The resulting solution (15 $\mu$l) was used in an amplification procedure as described in Example 1.

Water-insoluble probes, like those described in Example 1, were prepared and used containing four different oligonucleotides for the four alleles defined by the DNA sequences in the DQ$\alpha$ locus between codons 47 and 56 (Gyllensten and Erlich, Proc. Nat. Acad. Sci., USA, 85, pp. 7652-7656, 1988). The linker arm for each probe contained 28 ethylene glycol units. The four oligonucleotides had the following sequences wherein N represents the linking group described in Example 1:

SEQ ID NO: 4 5'-N-CTCAGGCCAC CGCCAGGCA-3'
SEQ ID NO: 5 5'-N-TTCCACAGAC TTAGATTTG-3'
SEQ ID NO: 6 5'-N-TTCCGCAGAT TTAGAAGAC-3'
SEQ ID NO: 7 5'-N-TGTTTGCCTG TTCTCAGAC-3'

The assay was carried as described in Example 1 using disposable test devices having microporous filtration membranes therein for separation. A reddish color was observed in the test devices in which probes labeled as SEQ ID NO: 5 and SEQ ID NO: 7 were used, indicating that the person from whom the hair was obtained has DNA segments in the HLA DG$\alpha$ locus corresponding to those probe sequences.

SPECIMEN SEQUENCE LISTING

SEQ ID NO: 1
SEQUENCE TYPE: Nucleotide
SEQUENCE LENGTH: 19 base pairs
STANDEDNESS: Single
TOPOLOGY: Linear
MOLECULE TYPE: Nucleic acid probe
IMMEDIATE SOURCE: Synthetically prepared
PROPERTIES: Complementary to human
HLA-DQ$\alpha$ obtained from the HLA homozygous cell line HAR, National Institute of General Medical Sciences Human Genetic Mutant Cell Repository, Coriell Institute for Medical Research, Camden, N.J. FEATURES: N represents an
aminotetraethyleneglycol linking arm having 16 ethylene glycol units

5'-N-CTGGTGTTTG CCTGTTCTC-3'

SEQ ID NO: 2
SEQUENCE TYPE: Nucleotide
SEQUENCE LENGTH: 26 base pairs
STANDEDNESS: Single
TOPOLOGY: Linear
MOLECULE TYPE: Nucleic acid primer
IMMEDIATE SOURCE: Synthetically prepared
PROPERTIES: Complementary to one strand
of human HLA-DQ$\alpha$ obtained from the homozygous cell line HAR, National Institute of General Medical Sciences Human Genetic Mutant Cell Repository, Coriell Institute for Medical Research, Camden, N.J.

FEATURES: N' represents a biotintetraethyleneglycol linking arm

5'-N'-GTGCTGCAGG TGTAAACTTG TACCAG-3'

SEQ ID NO: 3
SEQUENCE TYPE: Nucleotide
SEQUENCE LENGTH: 26 base pairs
STANDEDNESS: Single
TOPOLOGY: Linear
MOLECULE TYPE: Nucleic acid primer
IMMEDIATE SOURCE: Synthetically prepared
PROPERTIES: Complementary to one strand of human HLA-DQα obtained from the homozygous cell line HAR, National Institute of General Medical Sciences Human Genetic Mutant Cell Repository, Coriell Institute for Medical Research, Camden, N.J.
FEATURES: N' represents a biotintetraethyleneglycol linking arm

5'-N'-CGGATCCGGT AGCAGCGGTA GAGTTG-3'

SEQ ID NO: 4
SEOUENCE TYPE: Nucleotide
SEQUENCE LENGTH: 19 base pairs
STANDEDNESS: Single
TOPOLOGY: Linear
MOLECULE TYPE: Nucleic acid probe
IMMEDIATE SOURCE: Synthetically prepared
PROPERTIES: Complementary to a sequence between codons 47 and 56 of human HLA-DOα obtained from the homozygous cell line HAR, National Institute of General Medical Sciences Human Genetic Mutant Cell Repository, Coriell Institute for Medical Research, Camden, N.J. FEATURES: N represents an aminotetraethyleneglycol linking arm having 28 ethylene glycol units

5'-N'-CTCAGGCCAC CGCCAGGCA-3'

SEQ ID NO: 5
SEQUENCE TYPE: Nucleotide
SEQUENCE LENGTH: 19 base pairs
STANDEDNESS: Single
TOPOLOGY: Linear
MOLECULE TYPE: Nucleic acid probe
IMMEDIATE SOURCE: Synthetically prepared
PROPERTIES: Complementary to a sequence between codons 47 and 56 of human HLA-DQα obtained from the homozygous cell line HAR, National Institute of General Medical Sciences Human Genetic Mutant Cell Repository, Coriell Institute for Medical Research, Camden, N.J. FEATURES: N represents an aminotetraethyleneglycol linking arm having 28 ethylene glycol units

5'-N'-TTCCACAGAC TTAGATTTG-3'

SEQ ID NO: 6
SEQUENCE TYPE: Nucleotide
SEQUENCE LENGTH: 19 base pairs
STANDEDNESS: Single
TOPOLOGY: Linear
MOLECULE TYPE: Nucleic acid probe
IMMEDIATE SOURCE: Synthetically prepared
PROPERTIES: Complementary to a sequence

between codons 47 and 56 of human HLA-DQα obtained from the homozygous cell line HAR, National Institute of General Medical Sciences Human Genetic Mutant Cell Repository, Coriell Institute for Medical Research, Camden, N.J. FEATURES: N represents an
aminotetraethyleneglycol linking arm having 28 ethylene glycol units

5'-N'-TTCCGCAGAT TTAGAAGAC-3'

SEQ ID NO: 7
SEQUENCE TYPE: Nucleotide
SEQUENCE LENGTH: 19 base pairs
STANDEDNESS: Single
TOPOLOGY: Linear
MOLECULE TYPE: Nucleic acid probe
IMMEDIATE SOURCE: Synthetically prepared
PROPERTIES: Complementary to a Sequence
between codons 47 and 56 of human HLA-DQα obtained from the homozygous cell line HAR, National Institute of General Medical Sciences Human Genetic Mutant Cell Repository, Coriell Institute for Medical Research, Camden, N.J. FEATURES: N represents an
aminotetraethyleneglycol linking arm having 28 ethylene glycol units

5'-N'-TGTTTGCCTG TTCTCAGAC-3'

## Claims

1. A method for detecting HLA DNA comprising the steps of:
   A. amplifying HLA DNA strands in a specimen using a polymerase chain reaction and two primers complementary to the DNA strands, at least one of which primers is biotinylated, to form biotinylated primer extension products complementary to at least one of the strands,
   B. denaturing the amplified DNA strands and primer extension products in admixture,
   C. contacting the mixture with at least one oligonucleotide probe comprising a polymeric particle and an oligonucleotide, the oligonucleotide being
      (i) complementary to the biotinylated primer extension product,
      (ii) 15 to 25 nucleotides in length, and
      (iii) attached to the particle through a linking group having 16 to 30 ethylene glycol units
   to form an insoluble hybrid of the probe and biotinylated primer extension product,
   D. separating the insoluble hybrid from soluble materials,
   E. contacting the insoluble hybrid with a peroxidase-avidin conjugate to form a reaction product of biotinylated primer extension product and the conjugate,
   F. contacting the reaction product with a composition which provides a dye in the presence of hydrogen peroxide and peroxidase, and
   G. determining the dye formed in step F as a measure of the presence of HLA DNA in the specimen.

2. The method as claimed in claim 1 wherein a multiplicity of oligonucleotide probes are used, each having an oligonucleotide complementary to a different nucleic acid sequence of the biotinylated primer extension product.

3. The method as claimed in either of claims 1 and 2 wherein separation step D is carried out using a microporous membrane incorporated into a disposable test device.

4. A kit for detecting HLA DNA comprising:
   a. an oligonucleotide probe comprising a polymeric particle and an oligonucleotide, and
   b. a composition for providing a dye in the presence of peroxidase and hydrogen peroxide,
   the kit characterized wherein the oligonucleotide is
      (i) complementary to a HLA DNA strand,
      (ii) 15 to 25 nucleotides in length, and
      (iii) attached to the particle through a linking group having 16 to 30 ethylene glycol units.

5. The invention as claimed in any of claims 1 to 4 wherein the dye-providing composition comprises an imidazole leuco dye which provides a dye in the presence of hydrogen peroxide and peroxidase.

6. A kit for detecting HLA DNA comprising:
a. a oligonucleotide probe comprising a polymeric particle and an oligonucleotide, and
b. an avidin-peroxidase conjugate,
the kit characterized wherein the oligonucleotide is
(i) complementary to a HLA DNA strand,
(ii) 15 to 25 nucleotides in length, and
(iii) attached to the particle through a linking group having 16 to 30 ethylene glycol units.

7. The invention as claimed in any of claims 4 to 6 further comprising a disposable test device containing a microporous filtration membrane.

8. The invention as claimed in any of claims 4 to 7 further comprising the primers, dNTPs and DNA polymerase needed for DNA amplification.

9. The invention as claimed in any of claims 1 to 8 wherein the oligonucleotide of the probe has the following Sequence:

**SEQ ID NO: 1 5'-N-CTGGTGTTTG CCTGTTCTC-3'**

wherein N represents an aminotetraethylene glycol spacer arm.

10. The invention as claimed in any of claims 1 to 9 for the typing of DNA from the HLA-DQα locus.

11. The use of an oligonucleotide probe comprising a polymeric particle and an oligonucleotide, the oligonucleotide being
(i) complementary to a biotinylated primer extension product,
(ii) 15 to 25 nucleotides in length, and
(iii) attached to the particle through a linking group having 16 to 30 ethylene glycol units to detect HLA DNA.